# EUROPÄISCHE PATENTSCHRIFT

(11) **EP 3 681 551 B1**
(45) Veröffentlichungstag und Bekanntmachung des Hinweises auf die Patenterteilung: **15.07.2026**
(21) Anmeldenummer: 18780030.5
(22) Anmeldetag: 14.09.2018
(51) Int. Cl.: A61M 1/02, A61M 1/36

(54) **VORRICHTUNG ZUM ÜBERTRAGEN VON KÖRPERFLÜSSIGKEITEN**
DEVICE FOR TRANSFUSING BODY FLUIDS
DISPOSITIF PERMETTANT LE TRANSFERT DE LIQUIDES ORGANIQUES

(30) Priorität: 15.09.2017 DE 102017008657
(43) Veröffentlichungstag der Anmeldung: 22.07.2020
(73) Patentinhaber: Orthogen AG, 40212 Düsseldorf (DE)
(72) Erfinder: TROILLET, Julien, 04416 Markkleeberg (DE); REINECKE, Julio, 50733 Köln (DE); WEHLING, Peter, 40597 Düsseldorf (DE)
(74) Vertreter: Hoffmann Eitle
(86) Internationale Anmeldenummer: PCT/EP2018/074891
(87) Internationale Veröffentlichungsnummer: WO 2019/053193

(56) Entgegenhaltungen:
- DE-U- 6 801 269
- KR-A- 20120 107 255
- US-A- 3 013 557
- US-A1- 2014 205 514

## Beschreibung

Die vorliegende Erfindung betrifft eine Vorrichtung, ein Verfahren (nicht beansprucht) und eine Verwendung (nicht beansprucht) zum Übertragen von Körperflüssigkeiten.

Es ist bekannt, Körperflüssigkeiten zu handhaben, um diese zu prozessieren, insbesondere in einem Verfahrensschritt die Körperflüssigkeit zu zentrifugieren. Dazu muss die Körperflüssigkeit dem Körper entnommen und - ggf. nach dem Prozessieren, insbesondere einer Zentrifugation - wieder in eine andere Kammer oder dem Körper übertragen werden. Zum Übertragen von Körperflüssigkeiten, insbesondere Blut, sind Spritzen bekannt.

Eine Zentrifugation von Körperflüssigkeiten, die sich in einer Spritze befinden, ist bekannt, um beispielsweise einzelne Blutphasen zu separieren und deren Bereitstellung für therapeutische Zwecke zu ermöglichen. Insbesondere der Bereitstellung von mit Plättchen angereichertem Blutplasma (PRP) kommt hierbei große Bedeutung zu.

Bei der Zentrifugation kann Vollblut in einzelne Bestandteile aufgetrennt werden. Die Blutbestandteile können in Abhängigkeit von ihrer Dichte voneinander getrennt werden. Wird Vollblut einer Zentrifugation unterzogen, bilden sich in der Regel drei Phasen aus. Die unterste Schicht mit größter Dichte stellen die Erythrozyten dar, während die obere Schicht das von Zellen befreite Blutplasma ist. Zwischen der oberen und der unteren Schicht bildet sich eine dünne Schicht aus Leukozyten und Thrombozyten aus, der sogenannte Buffy Coat. Der Buffy Coat ist aufgrund der darin enthaltenen Thrombozyten von großem Interesse, da mit diesem eine induzierende Wirkung einer Vielzahl physiologischer Prozesse in Verbindung gebracht wird. Wichtig ist dabei, da es sich bei dem Buffy Coat um eine dünne Schicht handelt, diese sauber abzutrennen.

US 8,052,969 B2 beschreibt ein Verfahren und eine Verwendung des Verfahrens zur Herstellung von mit Plättchen angereichertem Blutplasma unter Verwendung einer 2-Kammer-Spritze. Bei der 2-Kammer-Spritze ist der Kolben einer ersten Spritze als eine weitere Spritze ausgestaltet. Nach einer Befüllung der ersten Spritze kann die gesamte 2-Kammer-Spritze einer Zentrifugation unterzogen werden, wobei sich die einzelnen Bestandteile des Blutes in Abhängigkeit ihrer Dichte voneinander trennen. In einem der Zentrifugation nachgelagerten Schritt kann mittels der weiteren Spritze die obere, Plättchen angereicherte Plasmaschicht von der unteren abgetrennt werden. Hierzu wird das Plasma in den Hohlraum der zweiten Spritze aufgezogen, wobei darauf geachtet werden muss, eine saubere Abtrennung des mit Plättchen angereicherten Blutplasmas von der Blutphase höherer Dichte zu erreichen. Danach kann die zweite Spritze von der ersten Spritze getrennt werden. Die durchzuführenden Verfahrensschritte erfordern einen geübten Anwender, wobei Kontaminationen sowohl von außen als auch mit Leuko- oder Erythrozyten nicht ausgeschlossen werden können. So ist ein Verlust bzw. eine verringerte Ausbeute an PRP durch erschwerte

Abtrennung von den Leuko- bzw. Erythrozyten möglich. Für einige weitere Beispiele aus dem relevanten Stand der Technik siehe US 2014/205514 A1, KR 2012 0107255 A, US 3 013 557 A, DE 68 01 269 U.

Vor diesem Hintergrund ist es eine Aufgabe der Erfindung, eine Vorrichtung, ein Verfahren und eine Verwendung bereitzustellen, wodurch die Bereitstellung einzelner Bestandteile von Körperflüssigkeiten ermöglicht wird bei möglichst geringem Probenverlust oder Verunreinigung der Bestandteile der Körperflüssigkeit.

Die Aufgabe wird gelöst durch den Gegenstand der unabhängigen Patentansprüche.

Vorteilhafte Ausführungsformen sind Gegenstand der abhängigen Patentansprüche und ergeben sich aus der nachfolgenden Beschreibung.

Die Erfindung geht von dem Grundgedanken aus, die Form einer Kammer, in der sich eine prozessierte Körperflüssigkeit befindet, derart auszugestalten, dass eine vereinfachte Abtrennung der Phasen einer Körperflüssigkeit auch für ungeübte Anwender ermöglicht wird. Die Form der Kammer ist derart gewählt, dass zwangsläufig eine vereinfachte Abtrennung beim Übertragen einzelner Phasen aus der Kammer ermöglicht wird, indem eine Langstreckung beim Übertragen der Phasen erfolgt, um die Grenzfläche zwischen den Phasen bei der Abtrennung zu minimieren und gleichzeitig eine Übertragung der einzelnen Phasen aus der Kammer zu schaffen, bei der die schon vorgenommene Separierung der einzelnen Phasen nicht gestört oder beeinflusst wird. Die Form der Kammer ermöglicht eine Bewegung der separierten Phasen, mit einer möglichst geringen Störung der Grenzflächen.

Die Körperflüssigkeit kann in der Kammer in einer Zentrifuge prozessiert werden. Die Vorrichtung ist damit geeignet in einer Zentrifuge eingesetzt zu werden. Die Vorrichtung kann derart ausgestaltet sein, um in mindestens zwei Lagen bzw. Stellungen in der Zentrifuge prozessiert zu werden. Die beiden Lagen unterscheiden sich durch eine 180°-Drehung der Vorrichtung quer zur Längsachse der Vorrichtung. Hierdurch kann erreicht werden, dass beispielsweise bei menschlichem oder tierischem Blut als Körperfluid die Erythrozyten "oben" oder "unten" nach dem Zentrifugieren angeordnet sind bezogen auf ein vorbestimmtes Ende der Vorrichtung. So ist es beispielsweise möglich, die Erythrozyten benachbart zu einem vorbestimmten Ende vorliegen zu haben oder den Buffy Coat mit den Platelets und Leukozyten an diesem Ende vorliegen zu haben.

Die Erfindung schafft eine Vorrichtung zum Übertragen von Körperfluid umfassend eine Kammer zur Aufnahme des Körperfluids, wobei die Kammer einen zylindrischen Abschnitt aufweist und sich in ihrem Endbereich verengt, wobei die Steigung der Wandung im Endbereich der Kammer entlang der Längsachse stetig ist.

Der vorgenannte Aspekt der Erfindung kann mit denfolgenden wiedergegebenen Merkmalen und weiteren Aspekten der Erfindung kombiniert werden.

Der Begriff "Körperfluid" im Sinne der Beschreibung umfasst Elemente des menschlichen oder tierischen Körpers, deren größte Anteile eine in flüssigen Aggregatzustand vorliegende Substanz darstellt. Der Begriff umfasst im Wesentlichen eine Flüssigkeit, wobei gasförmige Anteile nicht ausgeschlossen sein sollen. Der Begriff Körperfluid umfasst insbesondere Speichel, Lymphflüssigkeit, Urin, Knochenmark und bevorzugt Blut.

Der Begriff "Übertragung" im Sinne der Beschreibung umfasst die Aufnahme von Körperfluid, insbesondere in eine Kammer, und die spätere Abgabe von zumindest Teilen des Körperfluids, insbesondere aus der Kammer heraus.

Der Begriff "Kammer" im Sinne der Beschreibung umfasst einen Hohlraum, der das Körperfluid aufnehmen kann. Hierbei ist die Größe des Hohlraums variabel, wobei der Hohlraum eine maximale Größe haben kann und durch Bewegen einer Wandung die Größe verändert werden kann, um das enthaltene Fluid aus der Kammer zu drücken oder (nicht beansprucht) einzusaugen. Im Sinne der vorliegenden Beschreibung kann eine Kammer gegenüber der Umgebung geschlossen sein, wobei eine offene Kammer insbesondere nach Ausbildung einer sterilen Verbindung zum menschlichen oder tierischen Körper gebracht werden kann, um die Körperflüssigkeit aufzunehmen oder abzugeben.

Die Kammer kann vorbefüllt sein. Beispielsweise kann die Kammer in einem Bereich ein bioaktives Mittel aufweisen. Das bioaktive Mittel kann durch eine bioaktive Substanz oder eine Beschichtung in einem Bereich der Kammer realisiert sein. Die bioaktive Substanz und/oder die Beschichtung können für eine Modifikation eines in der Kammer befindlichen bzw. eingebrachten Körperfluids verwendet werden. Als bioaktive Substanz eignen sich insbesondere im Falle von menschlichem oder tierischem Blut als Körperfluid Antikoagulantien umfassend EDTA, Citrat, Heparin und/oder deren Derivate. Bei den bioaktiven Substanzen kann es sich auch um die innere Oberfläche der Kammer vergrößernde und/oder verändernde Substanzen handeln. Die innere Oberfläche verändernde und/oder vergrößernde Substanzen können eine induzierende Wirkung zur Bildung autologer Proteine haben. Die oberflächenverändernden und/oder oberflächenvergrößernden Substanzen umfassen Glasmehl, Glasgranulat, Quarzmehl, Quarzsand, Korund, Kügelchen, Perlen, Sand und Metalle. Die oberflächenverändernden und/oder oberflächenvergrößernden Substanzen umfassen auch organische Verbindungen und Polymere sowie biogene oder biologische Substanzen, wie beispielsweise Cellulose, Collagene, Alginate, Nukleinsäuren und andere von Zellen gebildete Proteine oder Metabolite. Die oberflächenverändernde und/oder oberflächenmodifizierende Substanz kann von im Wesentlichen fester, flüssiger oder gelartiger Konsistenz sein.

Der Begriff "zylindrischer Abschnitt" im Sinne der Beschreibung umfasst einen röhrenförmigen Abschnitt, der von einer Mantelfläche und zwei weiteren Begrenzungsflächen, die Schnittflächen definieren, eingeschlossen wird. Der "zylindrische Abschnitt" kann als eine ebene Kurve in einer Ebene dargestellt werden, die entlang einer Geraden, die nicht in der zuvor genannten Ebene enthalten ist, um eine vorgegebene Strecke verschoben wird. Die ebene Kurve ist an der Anfangs- und Endlage der Verschiebung eine der zuvor genannten Schnittflächen. Die ebene Kurve kann insbesondere ein Kreis, eine Ellipse, ein Polygon oder eine Kombination der zuvor genannten Formen sein. Besonders bevorzugt ist als zylindrischer Abschnitt im Sinne der Beschreibung ein senkrechter Kreiszylinder.

Der Begriff "Wandung" der Kammer umfasst eine innere der Kammer zugewandte Begrenzungsfläche, die insbesondere in Richtung der Längsachse des zylindrischen Abschnitts eine für das Abtrennen und/oder die Übertragung förderliche Form aufweisen kann.

Der Begriff "Steigung der Wandung" im Sinne der Beschreibung umfasst eine Veränderung des Abstands der Wandung von der Mittelachse einer Kammer bezogen auf den zylindrischen Abschnitt der Kammer. Die Steigung wird gegenüber dem Verlauf in Richtung der Mittellängsachse berücksichtigt und bestimmt. Es kann insbesondere die mathematische Steigung als Maß für die Steilheit einer Kurve ermittelt werden, wobei es für einen Aspekt der Erfindung wesentlich ist, dass die Steigung der Wandung stetig ist und damit im Sinne der Mathematik keine Sprünge vorliegen bzw. eine Änderung der Wandung kann beschränkt werden, indem sich auf hinreichend kleine Änderungen bei der Betrachtung eines Abstands entlang einer Längsachse der Kammer beschränkt werden kann. Die Steigung der Wandung kann insbesondere im Verlauf keine Diskontinuitäten aufweisen. Der Verlauf der Wandung außerhalb des "zylindrischen Abschnitts" kann bevorzugt ein exponentieller, polynomförmiger oder/oder hyperbolischer Verlauf sein. Ein linearer Verlauf der Wandung der Kammer außerhalb des "zylindrischen Abschnitts" ist nicht ausgeschlossen, sofern zudem auch ein anderer weiterer Verlauf, der exponentiell, polynomförmig oder/oder hyperbolisch sein kann, vorliegt.

Der Begriff "Endbereich" der Kammer umfasst in der vorliegenden Beschreibung einen an den zylindrischen Abschnitt unmittelbar anschließenden Bereich. Gemäß der vorliegenden Erfindung wird eine Form der Wandung einer Vorrichtung derart gewählt, dass sie sich sanft verändern kann, um keine Störungen bei dem Fließen der Körperflüssigkeit innerhalb der Kammer zu erzeugen. Insbesondere Sprünge in der Wandung außerhalb des zylindrischen Abschnitts können vermieden werden. Eine Verengung kann durchgeführt werden, um die Grenzfläche zwischen einzelnen separierten Phasen zu verkleinern und die einzelnen Volumina der separierten Phasen in Längsrichtung zu strecken. Das Körperfluid kann aus der Kammer übertragen werden, beispielsweise aus der Kammer herausgedrückt werden, mit möglichst wenigen Störungen und bei möglichst genauer Beobachtung des Orts der Grenzfläche zwischen einzelnen Phasen. Es ist auch möglich, dass ein Anteil einer Phase aus dem Endbereich herausgedrückt und/oder herausgesaugt werden kann und/oder herausgedrückt in einem nachfolgenden Schritt ein Anteil einer anderen oder derselben Phase mittels einer eingebrachten Kanüle oder Nadel werden kann. Insbesondere kann der Übergang vom zylindrischen Abschnitt zum Endbereich stetig ausgebildet sein, so dass keine Sprünge in der Steigung des Übergangs vorhanden sind

Der Endbereich kann bevorzugt rotationssymmetrisch zur Längsachse der Vorrichtung ausgestaltet sein, wobei eine rotationssymmetrische Ausgestaltung entlang der Längsachse der Vorrichtung fertigungstechnische Vorteile haben kann. Es kann aber auch in einer besonders bevorzugten Ausführungsform vorgesehen sein, dass der Endbereich nicht rotationssymmetrisch zur Längsachse der Vorrichtung ist. Insbesondere kann die Öffnung am Endbereich außermittig bezogen auf die Längsachse der Vorrichtung sein. Hierdurch kann eine angepasste Verjüngung erfolgen, bei der beispielsweise in einer Blickrichtung eine Wandung betrachtet werden kann, deren Steigung geringer oder höher ist als eine Steigung der Wandung quer zur Blickrichtung. Hierdurch können angepasste Bedingungen geschaffen werden.

In einer bevorzugten Ausführungsform ist die Länge des Endbereichs in der Erstreckung der Längsachse im Bereich von 18 mm bis 24 mm, bevorzugt 20 mm bis 22 mm, ganz besonders bevorzugt 21 mm. Das Volumen des Endbereichs ist dabei bevorzugt in einem Bereich von 4,1 ml +/- 10 %. Die Erstreckung des zylindrischen Abschnitts entlang der Längsachse ist dabei bevorzugt 54 mm bis 60 mm, besonders bevorzugt 55 mm bis 58 mm, ganz besonders bevorzugt 57 mm, wobei die Gesamtlänge der Vorrichtung zur Aufnahme des Körperfluids ungefähr 78 mm betragen kann. Das Volumen des zylindrischen Abschnitts beträgt im bevorzugten Ausführungsbeispiel 30 ml +/- 10 %.

In einer weiteren Ausführungsform kann die Erstreckung des Endbereichs entlang der Längsachse der Vorrichtung 27 mm bis 33 mm, bevorzugt 29 mm bis 31 mm, ganz besonders bevorzugt 30 mm, betragen. Das Volumen des Endbereichs kann dabei 5,6 ml +/- 10 % sein. Die Länge der Erstreckung des zylindrischen Abschnitts entlang der Längsachse kann dabei 44 mm bis 50 mm, bevorzugt 46 mm bis 48 mm und ganz besonders bevorzugt 47 mm, betragen, wobei die Gesamterstreckung der Vorrichtung entlang der Längsachse ungefähr 77 mm betragen kann. Das Volumen des zylindrischen Abschnitts kann 25,5 ml +/- 10 % betragen.

In der beanspruchten Ausführungsform weist ein Fluidvolumen in der Kammer mit einer Grundfläche, die einer Basisfläche des zylindrischen Abschnitts entspricht, im verengten Endbereich im Vergleich zu dem Fluidvolumen im zylindrischen Abschnitt eine mindestens 1,5 bis 2,0-fache Erstreckung entlang der Längsachse der Kammer auf. Der Begriff "Basisfläche" des zylindrischen Abschnitts umfasst eine Schnittfläche, die sich ergibt, wenn der zylindrische Abschnitt quer zur Längserstreckung geschnitten wird bzw. ist die Basisfläche die Fläche, aus der der zylindrische Abschnitt sich durch eine Parallelverschiebung dieser Fläche ergibt. Insbesondere kann die Basisfläche bei einem senkrechten Kreiszylinder ein Kreis sein. Die Grundfläche des betrachteten Fluids soll der Basisfläche entsprechen, um zum Ausdruck zu bringen, dass es sich bei dem betrachteten Fluidvolumen in der Kammer um ein Volumen handelt, das mindestens die Basisfläche des zylindrischen Abschnitts überdeckt und eine Ausdehnung hat entlang der Längsachse. Es wird quasi ein Referenzvolumen hinsichtlich seiner räumlichen Erstreckung entlang der Längsachse in zwei "Positionen" verglichen, wenn es im zylindrischen Abschnitt ist und zum anderen, wenn es im Bereich der Verengung ist.

In einer bevorzugten Ausführungsform ist der Verlauf der Wandung der Kammer im Endbereich zumindest abschnittsweise nicht linear, so dass zumindest ein Abschnitt vorliegt, der nicht gerade verläuft. Hierdurch kann eine möglichst hohe Steigung der Wandung erreicht werden.

In einer bevorzugten Ausführungsform weist der Endbereich eine Erstreckung entlang der Längsachse der Kammer im Bereich von 15 mm bis 40 mm auf und/oder der zylindrische Abschnitt eine Erstreckung entlang der Längsachse der Kammer im Bereich von 38 mm bis 63 mm auf. Hierdurch können Bereiche geschaffen werden, die eine besonders gute Beobachtung der Grenzfläche zwischen den einzelnen Phasen ermöglichen. Die Längserstreckung der einzelnen Phasen kann hierdurch erhöht werden, sofern auch der Querschnitt des entsprechenden Bereiches verringert wird.

In einer bevorzugten Ausführungsform weist die Wandung im Endbereich einen (a) exponentiellen (b) polynomförmigen oder (c) hyperbolischen Verlauf auf, wodurch ein stetiger Verlauf vorhanden sein kann, der zu keiner Störung der Grenzflächen zwischen den Phasen beiträgt, aber gleichzeitig auch eine Verringerung bzw. Verengung möglich ist, die über eine kurze Distanz der Längsachse zu einer großen Verengung führt. Der Verlauf der Wandung im Endbereich muss bezogen auf die Längsachse des Endbereichs bzw. der Vorrichtung nicht rotationssymmetrisch um die Längsachse gleich verlaufen, so dass sich auch ein nicht-rotationssymmetrischer Endbereich bezogen auf die Längsachse ergeben kann. Die Öffnung am Endbereich kann auf der Längsachse der Vorrichtung liegen, es kann aber auch vorgesehen sein, dass die Öffnung am Endbereich zur Längsachse verschoben vorliegt.

In einer bevorzugten Ausführungsform weist die Kammer eine starre Wandung auf, wodurch eine einfach handhabbare Vorrichtung geschaffen werden kann, die mit den Händen ergriffen werden kann, ohne dass es zu einer Störung der bereits separierten Phasen des Körperfluids kommen kann. Ferner kann eine Kammer mit einer starren Wandung in einer Zentrifuge eingebracht und beispielsweise mittels einer Stützung an der starren Wandung gelagert werden, um die Zentrifugation durchzuführen.

In der beanspruchten Ausführungsform sind eine Offnung am Endbereich der Kammer, die von dem zylindrischen Abschnitt beanstandet ist, und ein an der Öffnung außenseitig der Kammer angeordnetes Verbindungsstück vorhanden. Hierdurch kann das Körperfluid in die Kammer eingefüllt werden und nach einer erfolgten Prozessierung, insbesondere einer Zentrifugation, über die Öffnung aus der Kammer übertragen werden. Das Verbindungsstück ermöglicht eine vereinfachte Ausbildung einer Verbindung der Kammer mit einer weiteren Kammer und/oder dem menschlichen oder tierischen Körper.

Der Begriff "Verbindungsstück" beschreibt im Sinne der vorliegenden Beschreibung ein Bauteil, welches an der Vorrichtung vorhanden ist, um eine Verbindung der Vorrichtung mit externen Objekten durchzuführen. Die externen Objekte umfassen beispielsweise Kanülen, Infusionsschläuche, Spritzen und/oder weitere Kammern. Ein Verbindungsstück im Sinne der vorliegenden Beschreibung kann als ein Teil einer Schraub-, Luer-, Luer-Lock-, Bayonett- und/oder Steck-Verbindung ausgestaltet sein.

In einer bevorzugten Ausführungsform ist die Wandung der Kammer zumindest in einem Abschnitt des Endbereichs optisch durchsichtig ausgestaltet, um eine vereinfachte Sicht auf die Grenzfläche, die durch die Kammer bewegt wird, zu gewähren. Beispielsweise kann die Kammer, insbesondere im Endbereich, aus einem Kunststoff bestehen, der insbesondere in einem Abschnitt des Endbereichs durchsichtig ausgebildet ist. Kunststoffe können bei der Herstellung gut verarbeitet werden und können ein geringes Gewicht aufweisen.

In einer bevorzugten Ausführungsform weist die Kammer ein Volumen im Bereich von 5 bis 70 ml auf, um ein Körperfluid aufzunehmen. Derartige Größen von Kammern können insbesondere in einer Zentrifuge prozessiert werden, wobei die Größe auch ausreichend ist, dass eine Menge an einer interessierenden Phase erhalten werden kann.

Die Vorrichtung kann insbesondere als ein Hohlraum ausgestaltet sein, in dem sich ein beweglicher Kolben bewegen kann. An der dem Koben beabstandeten Seite des Hohlraums kann das Verbindungsstück in Form eines Luer-Lock-Verbindungsstücks vorliegen. Der Begriff "Kolben" im Sinne der Beschreibung umfasst dabei eine Wandung der Kammer, die in den Hohlraum geführt bewegt werden kann. An der beweglichen Wandung, die im Wesentlichen der Grundfläche des zylindrischen Abschnitts entsprechen kann, kann ein Stempel vorgesehen sein, mittels dem die Wandung verschoben werden kann. Zum Befüllen des Hohlraums kann die bewegliche Wandung mit dem Stempel gezogen werden, um den effektiven Hohlraum, in dem das Körperfluid aufgenommen werden kann, zu vergrößern. Die Körperflüssigkeit kann mittels der sich bewegenden Wandung in den Hohlraum eingesaugt werden. Zur vereinfachten Prozessierung der Vorrichtung in einer Zentrifuge kann ein an der bewegbaren Wandung angeordneter Stempel von der Wandung bzw. von einem an der Wandung angeordneten Verbindungselement getrennt werden. Beispielsweise kann der Stempel abgebrochen oder abgeschraubt werden. Die Vorrichtung bzw. Kammer kann in einer besonders bevorzugten Ausführungsform rotationssymmetrisch zur Längsachse ausgestaltet sein.

Um das Körperfluid bzw. den Kolben in der Kammer gut bewegen zu können, kann die Innenwandung des Hohlraums und/oder die Innenwand des Hohlraums kontaktierende Fläche des Kolbens glatt ausgestaltet sein. Ferner kann bei dem Zusammenwirken von Kolben und Innenwandung des Hohlraums eine besonders ruhige Bewegung ausgeführt werden, die zu keiner Störung der Grenzflächen des Körperfluids führt. Insbesondere kann der innere Hohlraum und der Kolben derart ausgestaltet sein, dass Reibung minimiert ist. Dies kann durch eine geeignete Materialwahl und/oder Beschichtung der entsprechenden Kontaktfläche(n) geschehen.

Die Erfindung schafft auch ein Verfahren (nicht beansprucht) zum Übertragen von Körperfluid umfassend
eine Kammer, wobei in der Kammer das Körperfluid aufgenommen wird und die Kammer einen zylindrischen Abschnitt aufweist und sich in einem Endbereich verengt, wobei die Steigung der Wandung der Kammer im Endbereich entlang der Längsachse stetig ist und insbesondere ein prozessiertes Körperfluid aus der Kammer über den Endbereich ausgelassen wird.

Die Erfindung schafft auch eine Verwendung (nicht beansprucht) einer Kammer beim Ubertragen eines

Körperfluids, wobei die Kammer zur Aufnahme des Körperfluids verwendet wird, wobei die Kammer einen zylindrischen Abschnitt aufweist und sich in ihrem Endbereich verengt und wobei die Steigung der Wandung im Endbereich der Kammer entlang der Längsachse stetig ist, wobei diese Kammer insbesondere verwendet wird, prozessiertes Körperfluid zu trennen.

Die Erfindung wird nachfolgend anhand von Zeichnungen, die eine Ausführungsform der Erfindung zeigen, näher erläutert. Darin zeigt:
- Fig. 1:: eine schematische Darstellung einer Blut enthaltenen Vorrichtung vor einer Zentrifugation;
- Fig. 2:: die Vorrichtung gemäß Figur 1 nach einer Zentrifugation
- Fig. 3:: die Vorrichtung gemäß Figur 2 nach einem Übertragen eines Teils des Körperfluids aus der Kammer;
- Fig. 4:: einen Verlauf einer Wandung entlang einer Längsachse; und
- Fig. 5:: eine weitere Ausführungsform.

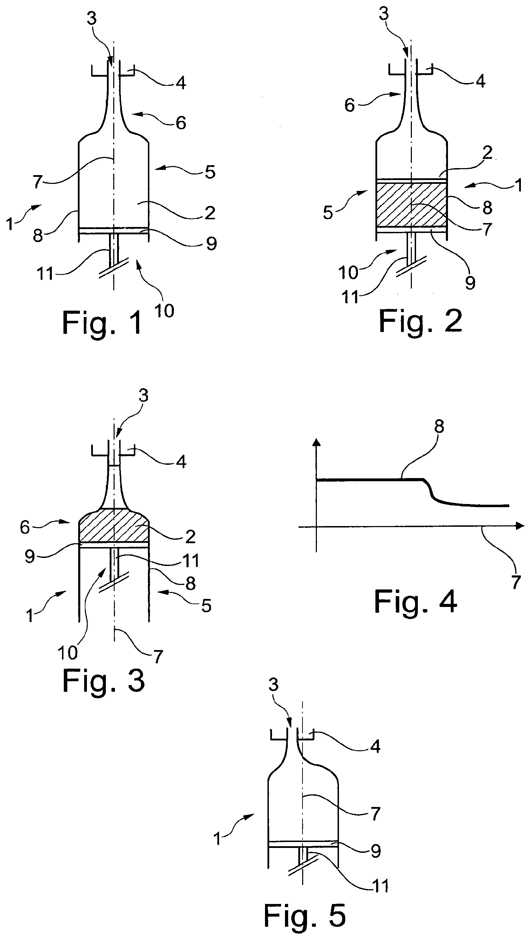

Figur 1 zeigt eine schematische Darstellung einer Blut enthaltenden Vorrichtung 1. Die Vorrichtung 1 weist eine Kammer 2 zur Aufnahme des Blutes auf. Die Vorrichtung weist ferner eine Öffnung 3 auf, die in der Figur 1 oben angeordnet ist. Mittels der Öffnung 3 kann Blut in die Kammer 2 hineingesaugt oder aus der Kammer 2 abgeleitet werden. Endseitig an der Vorrichtung 1 ist benachbart zur Öffnung 3 ein Verbindungsstück 4 angeordnet, welches einstückig mit der Vorrichtung 1 ausgebildet ist. Das Verbindungsstück 4 ist im dargestellten Fall in Form eines Luer-Lock-Verbindungselements ausgestaltet.

Die Kammer 2 weist einen zylindrischen Abschnitt 5 und einen sich direkt an den zylindrischen Abschnitt 5 anschließenden Endbereich 6 auf, mittels dem eine Verengung des Querschnitts der Kammer in Richtung der Öffnung 3 erfolgt.

Im dargestellten Ausführungsbeispiel der Figur 1 ist der zylindrische Abschnitt 5 ein gerader Kreiszylinder und die Kammer 2 rotationssymmetrisch ausgeführt. Es liegt eine Achsensymmetrie und Rotationssymmetrie zur Längsachse 7 vor. Die Wandung der Kammer 2 verläuft im zylindrischen Abschnitt 5 linear und im Übergang zum Endbereich 6 sowie im Endbereich 6 selbst stetig ohne einen Sprung. Betrachtet man den Abstand zwischen der Wandung 8 der Kammer 2 und der Längsachse 7 als Funktionswert über der Längsachse 7 (vgl. Figur 4), so kann das Fehlen von Sprüngen in dem Verlauf der Wandung 8 dadurch beschrieben werden, dass die Ableitung bzw. Steigung der Funktion über der Längsachse 7 stetig verläuft.

Die Vorrichtung 1 weist ferner eine Platte 9 auf, die im Wesentlichen die Abmessung der Basisfläche des zylindrischen Abschnitts 5 hat. Die Platte 9 ist als Teil eines Kolbens 10 mit einem Stempel 11 ausgeführt. Mit der Bewegung der Platte 9 kann das effektive Volumen in der Kammer 2 verringert bzw. vergrößert werden. Beispielsweise kann die Platte 9 von der Öffnung 3 weggezogen werden und Blut in die Kammer 2 eingesaugt werden. In dem in Figur 1 dargestellten Ausführungsbeispiel kann der Stempel 11 von der Platte 9 getrennt werden, um ein Prozessieren der Vorrichtung 1 in einer Zentrifuge vereinfacht zu ermöglichen. Die Längsausdehnung der Vorrichtung 1 kann durch das Entfernen des Stempels 11 von der Platte 9 verringert werden.

In Figur 2 ist der Zustand dargestellt, der sich nach der Zentrifugation von Vollblut einstellt. Die obere Schicht bzw. Phase ist das Plasma und die untere Schicht sind die Erythrozyten. Zwischen dem Plasma und den Erythrozyten ist der sogenannte Buffy Coat angeordnet, eine dünne Schicht aus Leukozyten und Thrombozyten.

In Figur 3 ist der Zustand dargestellt, nach dem die Platte 9 auf die Öffnung 3 zubewegt wurde und ein Teil des Plasmas aus der Kammer 2 herausgedrückt oder herausgesaugt wurde. Durch die stetigen Übergänge und den stetigen Verlauf des zylindrischen Abschnitts 5, und dem Übergang zwischen dem zylindrischen Abschnitt 5 und dem Endbereich 6 und dem stetigen Verlauf im Endbereich 6 selbst, erfolgt so gut wie keine Störung der Grenzflächen zwischen den einzelnen Phasen und durch die Verengung im Endbereich 6 wird eine Streckung des Volumens erreicht, so dass wie in Figur 3 zu sehen ist, der Buffy Coat in die Länge gestreckt wird und die Grenzfläche als möglichst scharfe Grenzfläche zwischen Buffy Coat und Erythrozyten erhalten bleibt. Bei dem in Figur 3 dargestellten Beispiel ist eine Entnahme beispielsweise mittels einer 10 ml Spritze möglich, die den Buffy Coat aufnehmen kann.

Figur 4 zeigt schematisch die Wandung 8 der Kammer 2 sowohl im zylindrischen Abschnitt 5 als auch im Endbereich 6 als Funktion über der Längsachse 7. Die Wandung verläuft stetig und insbesondere die Steigung der Funktion weist einen stetigen Verlauf auf, wobei hierdurch eine Funktion beschrieben wird, die keine Sprünge aufweist und insbesondere keine Diskontinuitäten, die zu einer Störung der Grenzfläche führen könnten.

Die Figur 5 zeigt schematisch eine weitere Ausführungsform einer Vorrichtung, die der zuvor beschriebenen Ausführungsform der Vorrichtung gleicht, wobei im Endbereich 6 keine Rotationssymmetrie der Wandung zur Längsachse 7 vorliegt.

## Patentansprüche

1. Vorrichtung (1) zum Übertragen von Körperfluid umfassend eine Kammer (2) zur Aufnahme des Körperfluids, wobei die Kammer (2) einen zylindrischen Abschnitt (5) aufweist und sich in ihrem Endbereich (6) verengt, und eine Öffnung (3) am Endbereich der Kammer (2), die von dem zylindrischen Abschnitt beabstandet ist, und ein an der Öffnung (3) außenseitig der Kammer (2) angeordnetes Verbindungsstück (4) vorgesehen ist, wobei die Kammer (2) einen in der Größe variablen Hohlraum, der das Körperfluid aufnehmen kann, und eine Wandung aufweist und die Wandung derart beweglich ist, dass mittels Bewegen der Wandung die Größe des Hohlraums der Kammer (2) veränderbar ist, um in der Kammer (2) enthaltenes Körperfluid aus der Kammer (2) zu drücken, **dadurch gekennzeichnet, dass** die Form der Kammer derart gewählt ist, dass zwangsläufig eine vereinfachte Abtrennung beim Übertragen einzelner Phasen des Körperfluids aus der Kammer ermöglicht wird, indem eine Langstreckung beim Übertragen der Phasen erfolgt, um die Grenzfläche zwischen den Phasen bei der Abtrennung zu minimieren und gleichzeitig eine Übertragung der einzelnen Phasen aus der Kammer zu schaffen, bei der die schon vorgenommene Separierung der einzelnen Phasen nicht gestört oder beeinflusst wird, wobei die Steigung der Wandung (8) im Endbereich der Kammer (2) entlang der Längsachse (7) stetig ist, sodass ein Fluidvolumen in der Kammer (2) mit einer Grundfläche, die einer Basisfläche des zylindrischen Abschnitts entspricht, im verengten Endbereich (6) im Vergleich zum gleichen Fluidvolumen im zylindrischen Abschnitt (5) eine mindestens 1,5 bis 2,0-fache Erstreckung entlang der Längsachse (7) der Kammer (2) aufweist.

2. Vorrichtung (1) nach Anspruch 1, **dadurch gekennzeichnet, dass** die Wandung (8) der Kammer (2) im Endbereich (6) zumindest abschnittsweise nicht linear verläuft.

3. Vorrichtung (1) nach einem der Ansprüche 1 oder 2, **dadurch gekennzeichnet, dass** der Endbereich (6) eine Erstreckung entlang der Längsachse (7) der Kammer (2) im Bereich von 15 mm bis 40 mm aufweist und/oder der zylindrische Abschnitt (5) eine Erstreckung entlang der Längsachse (7) der Kammer (2) im Bereich von 38 mm bis 63 mm aufweist.

4. Vorrichtung (1) nach einem der Ansprüche 1 bis 3, wobei die Wandung (8) im Endbereich (6) einen (a) exponentiellen, (b) polynomförmigen und/oder (c) hyberbolischen Verlauf aufweist.

5. Vorrichtung (1) nach einem der Ansprüche 1 bis 4, wobei die Kammer (2) eine starre Wandung (8) aufweist.

6. Vorrichtung (1) nach einem der Ansprüche 1 bis 5, wobei das Verbindungsstück (4) als Teil einer (a) Schraubverbindung, (b) Luer- oder Luer-Lock-Verbindung, (c) Bajonett-Verbindung und/oder (d) Steckverbindung ausgestaltet ist.

7. Vorrichtung (1) nach einem der Ansprüche 1 bis 6, wobei die Wandung (8) der Kammer (2) zumindest in einem Abschnitt des Endbereichs (6) optisch durchsichtig ausgestaltet ist.

8. Vorrichtung (1) nach einem der Ansprüche 1 bis 7, wobei die Kammer (2) ein Volumen im Bereich von 5 bis 50 ml aufweist.

## Claims

1. Device (1) for transferring body fluid comprising a chamber (2) for receiving the body fluid, wherein the chamber (2) has a cylindrical section (5) and narrows in its end region (6), and an opening (3) at the end region of the chamber (2), which is spaced apart from the cylindrical section, and providing for a connecting piece (4) arranged at the opening (3) outside the chamber (2), wherein the chamber (2) has a variable-size cavity, which can receive the body fluid, and a wall, and the wall is movable in such a way that by means of moving the wall, the size of the cavity of the chamber (2) can be changed in order to force body fluid contained in the chamber (2) out of the chamber (2), **characterised in that** the shape of the chamber is selected in such a way that a simplified separation is inevitably made possible when transferring individual phases of the body fluid from the chamber, **in that** a long extension is made when transferring the phases, in order to minimise the interface between the phases during the separation and at the same time create a transferring of the individual phases from the chamber, in which the separation already made of the individual phases is not disturbed or influenced, wherein the slope of the wall (8) is continuous in the end region of the chamber (2) along the longitudinal axis (7), such that a fluid volume in the chamber (2) having a surface area corresponding to a base area of the cylindrical section, has, in the narrowed end region (6), at least 1.5 to 2.0 times the extent along the longitudinal axis (7) of the chamber (2) compared to the same fluid volume in the cylindrical section (5).

2. Device (1) according to claim 1, **characterised in that** the wall (8) of the chamber (2) extends non-linearly at least in sections in the end region (6).

3. Device (1) according to one of the claims 1 or 2, **characterised in that** the end region (6) has an extent along the longitudinal axis (7) of the chamber (2) in the range of 15 mm to 40 mm and/or the cylindrical section (5) has an extent along the longitudinal axis (7) of the chamber (2) in the range of 38 mm to 63 mm.

4. Device (1) according to any of claims 1 to 3, wherein the wall (8) in the end region (6) has an (a) exponential, (b) polynomial and/or (c) hyperbolic profile.

5. Device (1) according to any of the claims 1 to 4, wherein the chamber (2) has a rigid wall (8).

6. Device (1) according to any of claims 1 to 5, wherein the connecting piece (4) is configured as part of an (a) screw connection, (b) Luer or Luer lock connection, (c) bayonet connection and/or (d) plug connection.

7. Device (1) according to any of claims 1 to 6, wherein the wall (8) of the chamber (2) is optically transparent at least in one section of the end region (6).

8. Device (1) according to any of claims 1 to 7, wherein the chamber (2) has a volume in the range of 5 to 50 ml.

## Revendications

1. Dispositif (1) permettant le transfert de liquides organiques et comprenant une chambre (2) pour la réception des liquides organiques, dans lequel la chambre (2) comporte une section cylindrique (5) et se rétrécit dans sa zone d'extrémité (6), et une ouverture (3) est prévue dans la zone d'extrémité de la chambre (2), ouverture qui est disposée à distance de la section cylindrique, et un élément de raccordement (4) disposé au niveau de l'ouverture (3) à l'extérieur de la chambre (2), dans lequel la chambre (2) comporte une cavité de taille variable pouvant recevoir les liquides organiques et une paroi, la paroi étant mobile de telle sorte que, par déplacement de ladite paroi, la taille de la cavité de la chambre (2) peut être modifiée afin d'expulser de la chambre (2) les liquides organiques contenus dans la chambre (2), **caractérisé en ce que** la forme de la chambre est choisie de manière à permettre nécessairement une séparation simplifiée lors du transfert des phases individuelles des liquides organiques hors de la chambre, une extension longitudinale intervenant lors du transfert des phases afin de minimiser l'interface entre les phases lors de la séparation et assurer simultanément un transfert des phases individuelles hors de la chambre sans perturber ni influencer la séparation déjà effectuée desdites phases individuelles, dans lequel la pente de la paroi (8) dans la zone d'extrémité de la chambre (2) le long de l'axe longitudinal (7) est continue, de sorte qu'un volume de liquide organique dans la chambre (2) avec une surface de base correspondant à une surface de base de la section cylindrique, présente dans la zone d'extrémité rétrécie (6), par rapport au même volume de liquide organique dans la section cylindrique (5), une extension le long de l'axe longitudinal (7) de la chambre (2) au moins 1,5 à 2,0 fois plus importante.

2. Dispositif (1) selon la revendication 1, **caractérisé en ce que** la paroi (8) de la chambre (2) s'étend de manière non linéaire au moins sur une portion dans la zone d'extrémité (6).

3. Dispositif (1) selon l'une des revendications 1 ou 2, **caractérisé en ce que** la zone d'extrémité (6) présente une extension le long de l'axe longitudinal (7) de la chambre (2) dans une plage de 15 mm à 40 mm et/ou la section cylindrique (5) présente une extension le long de l'axe longitudinal (7) de la chambre (2) dans une plage de 38 mm à 63 mm.

4. Dispositif (1) selon l'une quelconque des revendications 1 à 3, dans lequel la paroi (8) présente dans la zone d'extrémité (6) un tracé (a) exponentiel, (b) polynomial et/ou (c) hyperbolique.

5. Dispositif (1) selon l'une quelconque des revendications 1 à 4, dans lequel la chambre (2) présente une paroi rigide (8).

6. Dispositif (1) selon l'une quelconque des revendications 1 à 5, dans lequel l'élément de raccordement (4) est conçu comme faisant partie d'un (a) raccord vissé, (b) raccord Luer ou Luer-Lock, (c) raccord à baïonnette et/ou (d) raccord enfichable.

7. Dispositif (1) selon l'une quelconque des revendications 1 à 6, dans lequel la paroi (8) de la chambre (2) est conçue de manière optiquement transparente au moins dans une portion de la zone d'extrémité (6).

8. Dispositif (1) selon l'une quelconque des revendications 1 à 7, dans lequel la chambre (2) présente un volume compris dans une plage de 5 à 50 ml.
